(19) **Europäisches Patentamt / European Patent Office / Office européen des brevets**

(11) **EP 4 120 279 A1**

(12) # EUROPEAN PATENT APPLICATION
published in accordance with Art. 153(4) EPC

(43) Date of publication:
**18.01.2023 Bulletin 2023/03**

(21) Application number: **20928847.1**

(22) Date of filing: **14.05.2020**

(51) International Patent Classification (IPC):
***G16B 30/10*** (2019.01)

(86) International application number:
**PCT/CN2020/090175**

(87) International publication number:
**WO 2021/196356 (07.10.2021 Gazette 2021/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **02.04.2020 CN 202010254690**

(71) Applicant: **Shanghai Zj Bio-tech Co., Ltd.**
**Pudong, Shanghai 201114 (CN)**

(72) Inventors:
• **JI, Cong**
**Shanghai 201114 (CN)**
• **SHAO, Junbin**
**Shanghai 201114 (CN)**
• **LIU, Yan**
**Shanghai 201114 (CN)**
• **QI, Xia**
**Shanghai 201114 (CN)**
• **JIN, Yudan**
**Shanghai 201114 (CN)**
• **LI, Qiteng**
**Shanghai 201114 (CN)**

(74) Representative: **Canzler & Bergmeier**
**Patentanwälte**
**Partnerschaft mbB**
**Despag-Straße 6**
**85055 Ingolstadt (DE)**

(54) **METHOD AND APPARATUS FOR IDENTIFYING MULTI-COPY REGION IN MICROBIAL TARGET FRAGMENT, AND USE**

(57) A method for identifying a multi-copy region in a microbial target fragment. The method comprises at least the following steps: searching for a candidate multi-copy region: performing an internal comparison on a microbial target fragment, and searching for a region corresponding to a sequence to be tested, the similarity of which meets a preset value, to serve as a candidate multi-copy region, wherein the similarity refers to the product of the coverage rate and the matching rate of the sequence to be tested (S1); and obtaining a multi-copy region by means of verification: obtaining the median of a copy number of the candidate multi-copy region, and if the median of the copy number of the candidate multi-copy region is greater than one, recording the candidate multi-copy region as a multi-copy region (S2). The method for identifying a multi-copy region in a microbial target fragment is high in accuracy and sensitivity; an undiscovered multi-copy region can be identified; repetitive sequences can be found in an incompletely assembled motif; and compared with a method using 16S rRNA, the method is more comprehensive.

FIG. 1

## Description

### Technical Field

The present disclosure relates to the field of bioinformatics, and in particular, to a method and a device for identifying multi-copy regions in microorganism target fragments and a use thereof.

### Background

DNA concentrations of pathogenic microorganisms in biological samples are mostly very low and close to the detection limit. Traditional Polymerase Chain Reaction (PCR) or real-time PCR is often lack of detection sensitivity. Other methods such as two-step nested PCR may have better sensitivity. However, these methods are time-consuming, costly, and have poor accuracy. Therefore, it is important to improve the detection sensitivity. One way is to find a suitable template region when designing primers and probes. Usually, plasmids and 16S rRNA are used.

However, using plasmids for primer design would cause some problems: Not all microorganisms contain species-specific plasmids. Some microorganisms even have no plasmids. First of all, the species specificity of plasmid DNA is uncertain. The sequences on plasmids of some species are highly similar to those on plasmids of other species. Therefore, plasmid-based PCR tests are at a high risk of producing false positive or false negative results. Many clinical laboratories still need to use other PCR primer pairs for confirmatory experiments. Secondly, plasmids are not universal. Some species do not have plasmids, so it is not possible to use plasmids to detect the species, let alone to design primers and probes on plasmids to improve the detection sensitivity. For example, it has been reported that about 5% of Neisseria gonorrhoeae strains cannot be detected since they lack plasmids in recent studies.

Similarly, using rRNA gene regions as templates for PCR detection also has some problems: although rRNA genes exist in the genomes of all microbial species, and there are often multiple copies that can improve detection sensitivity. In fact, not all rRNA genes are multiple copies. For example, there is only one copy of rRNA gene in mycobacterium tuberculosis H37Rv. In addition, some changes in rRNA gene sequence are not suitable for detection. For example, between closely related species or even between strains of different subtypes of the same species, rRNA genes cannot meet the requirements of species specificity or even sub-species specificity because the sequence of rRNA genes is too conservative.

### Summary

The present disclosure provides a method and a device for identifying multi-copy regions in microorganism target fragments and a use thereof.

A first aspect of the present disclosure provides a method for identifying multi-copy regions in microorganism target fragments, which includes at least the following operations:

S100, searching for a candidate multi-copy region: performing an internal alignment on a microorganism target fragment, and searching for a region corresponding to a to-be-detected sequence of which a similarity meets a preset value as the candidate multi-copy region, the similarity being a product of a coverage rate and a matching rate of the to-be-detected sequence;

S200, verifying and obtaining a multi-copy region: obtaining a median value of the copy numbers of the candidate multi-copy region; if the median value of the copy numbers of the candidate multi-copy region is greater than 1, the candidate multi-copy region is recorded as a multi-copy region.

A second aspect of the present disclosure provides a device for identifying multi-copy regions in microorganism target fragments, which includes at least the followings:

a candidate multi-copy region searching module, configured to perform internal alignment on a microorganism target fragment, and search for a region corresponding to a to-be-detected sequence of which a similarity meets a preset value as a candidate multi-copy region, the similarity being a product of a coverage rate and a matching rate of the to-be-detected sequence;

a multi-copy region verifying and obtaining module, configured to obtain a median value of copy numbers of the candidate multi-copy region; if the median value of the copy numbers of the candidate multi-copy region is greater than 1, the candidate multi-copy region is recorded as a multi-copy region.

A third aspect of the present disclosure provides a computer readable storage medium, which stores a computer program. When executed by a processor, the program implements the above-mentioned method for identifying multi-copy regions in microorganism target fragments.

A fourth aspect of the present disclosure provides a computer processing device, including a processor and the above-mentioned computer readable storage medium. The processor executes the computer program on the computer readable storage medium to implement the operations of the above-mentioned method for identifying multi-copy regions in microorganism target fragments.

A fifth aspect of the present disclosure provides an electronic terminal, including a processor, a memory and a communicator; the memory stores a computer program, the communicator communicates with an external device, and the processor executes the computer program stored in the memory, so that the electronic terminal executes the above-mentioned method for identifying multi-copy regions in microorganism target fragments.

A sixth aspect of the present disclosure provides a use of the above-mentioned method for identifying multi-copy regions in microorganism target fragments, the above-mentioned device for identifying multi-copy regions in microorganism target fragments, the above-mentioned computer readable storage medium, the above-mentioned computer processing device or the above-mentioned electronic terminal in the detection of multi-copy regions in microorganism target fragments.

As described above, the method and the device for identifying multi-copy regions in microorganism target fragments and the use thereof according to the present disclosure have the following beneficial effects:

Compared with a literature database, the method for identifying multi-copy regions in microorganism target fragments is high in accuracy and high in sensitivity, and an undiscovered multi-copy region can be identified; a repetitive sequence can be found in incompletely assembled motifs; the method is more comprehensive than 16srRNA, which is not always multi-copy. The system is not limited to whether there is a whole genome sequence. Operational tasks can be submitted by providing the names of the target strains and comparison strains or by uploading sequence files locally.

## Brief Description of the Drawings

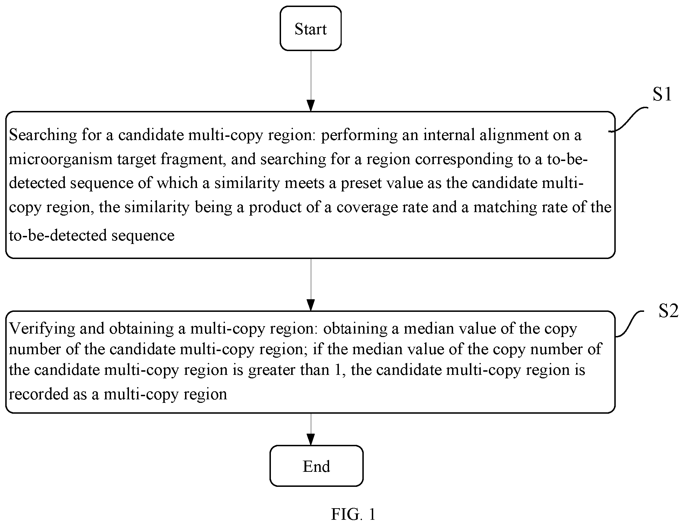

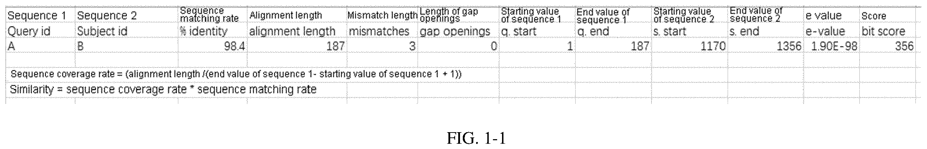

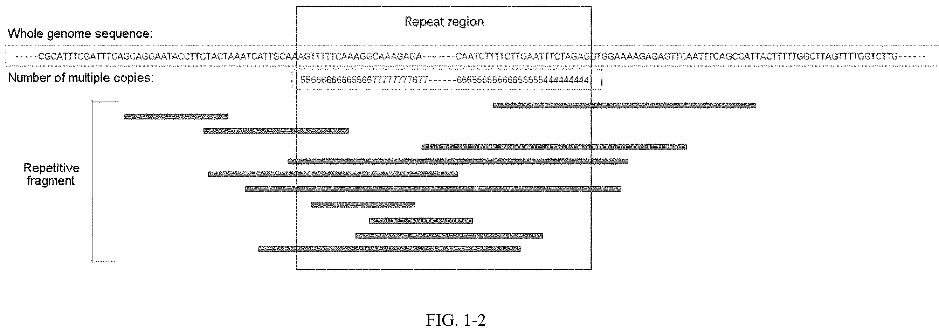

FIG. 1 is a flow chart of the method according to an embodiment of the present disclosure.
FIG. 1-1 is a graph showing calculation results of the coverage rate and sequence matching rate of aligned sequences.
FIG. 1-2 is a schematic diagram of the multi-copy region verifying and obtaining module according to the present disclosure.
FIG. 2 is a schematic diagram of the device according to an embodiment of the present disclosure.
FIG. 3 is a schematic diagram of the electronic terminal according to an embodiment of the present disclosure.

## Detailed Description of the Preferred Embodiments

The embodiments of the present disclosure will be described below. Those skilled in the art can easily understand other advantages and effects of the present disclosure according to contents disclosed by the specification. The present disclosure may also be implemented or applied through other different specific implementation modes. Various modifications or changes may be made to all details in the specification based on different points of view and applications without departing from the spirit of the present disclosure.

In addition, it should be understood that one or more method operations mentioned in the present disclosure are not exclusive of other method operations that may exist before or after the combined operations or that other method operations may be inserted between these explicitly mentioned operations, unless otherwise stated. It should also be understood that the combined connection relationship between one or more operations mentioned in the present disclosure does not exclude that there may be other operations before or after the combined operations or that other operations may be inserted between these explicitly mentioned operations, unless otherwise stated. Moreover, unless otherwise stated, the numbering of each method step is only a convenient tool for identifying each method step, and is not intended to limit the order of each method step or to limit the scope of the present disclosure. The change or adjustment of the relative relationship shall also be regarded as the scope in which the present disclosure may be implemented without substantially changing the technical content.

Please refer to FIG. 1 to FIG. 3. It needs to be stated that the drawings provided in the following embodiments are just used for schematically describing the basic concept of the present disclosure, thus only illustrating components only related to the present disclosure and are not drawn according to the numbers, shapes and sizes of components during actual implementation, the configuration, number and scale of each components during actual implementation thereof may be freely changed, and the component layout configuration thereof may be more complicated.

As shown in FIG. 1, the method for identifying multi-copy regions in microorganism target fragments according to the present disclosure includes at least the followings:

S100, searching for a candidate multi-copy region: performing an internal alignment on a microorganism target fragment, and searching for a region corresponding to a to-be-detected sequence of which a similarity meets a preset value as the candidate multi-copy region, the similarity being a product of a coverage rate and a matching rate of the to-be-detected sequence;

S200, verifying and obtaining a multi-copy region: obtaining a median value of the copy numbers of the candidate multi-copy region; if the median value of the copy numbers of the candidate multi-copy region is greater than 1, the candidate multi-copy region is recorded as a multi-copy region.

The preset value of the similarity may be determined as needed. The recommended preset value of the similarity should exceed 80%, such as 85%, 90%, 95%, 96%, 97%, 98%, 99%, or 100%.

The coverage rate = (length of similar sequence/(end value of the to-be-detected sequence - starting value of the to-be-detected sequence + 1))%.

The matching rate refers to the identity value when the to-be-detected sequence is aligned with another sequence. The identity value of the two aligned sequences may be obtained by softwares such as needle, water or blat.

The length of similar sequences refers to the number of bases that the matched fragment occupies in the to-be-detected sequence when the to-be-detected sequence is aligned with another sequence, that is, the length of the matched fragment.

For example, the data situation of a to-be-detected sequence corresponding to a candidate multi-copy region is shown in FIG. 1-1.

Sequence A is the to-be-detected sequence; when sequence A is aligned with sequence B, the length of the matched fragment is 187, the starting value (i.e., the starting position) of sequence A is 1, and the end value (i.e., the ending position) is 187, then:

$$\text{Coverage rate of sequence A} = (187/(187-1+1))*100\% = 100\%.$$

The matching rate of sequence A and sequence B corresponds to an identity of 98.4%.

Then the similarity between A and B=100%*98.4%=98.4%. The similarity preset value is set to 80%. The similarity between A and B satisfies the preset value. Therefore, A and B serve as candidate multi-copy regions.

The positions of the bases between the two to-be-aligned sequences do not cross (that is, the two aligned sequences are separated in the microorganism target fragment, and there is no overlapping part). The aligned sequence pair with regional overlapping may be removed before or after the alignment to obtain the similarity value. For example, as shown in FIG. 1, the positions of the bases in sequence B will not appear between 1-187 if the position of sequence A is 1-187. After the coverage rate and match rate are calculated, the uniq function may be used for de-duplication.

In operation S200, the obtaining of the median value of the copy numbers of the candidate multi-copy region includes: determining the position of each candidate multi-copy region on the microorganism target fragment, obtaining the number of other candidate multi-copy regions covering the position of each base of the to-be-verified candidate multi-copy region, and calculating the median value of the copy numbers of the to-be-verified candidate multi-copy region. The above-mentioned other candidate multi-copy regions refer to candidate multi-copy regions other than the to-be-verified candidate multi-copy region.

Specifically, for example, as shown in FIG. 1-2, the first row represents the sequence of the microorganism target fragment. In the sequence of the microorganism target fragment, the fragment within the frame is the to-be-verified candidate multi-copy region. The number in the second row is the number of multiple copies corresponding to each base in the to-be-verified candidate multi-copy region. The gray fragments in the figure represent the candidate multi-copy regions other than the to-be-verified candidate multi-copy region (hereinafter referred to as repetitive fragments). From the left to the right, the first base A in the first row of the frame appears in 5 repetitive fragments (that is, covered by 5 repetitive fragments). Therefore, it is considered that the number of repetitive fragments corresponding to the position of the first base A is 5, then the number of multiple copies at this position is 5. Take the last base G in the frame in the figure as another example, the number of repetitive fragments corresponding to the position of the last base G is 4, that is, the number of multiple copies at this position is 4. By analogy, the number of repetitive fragments covering the position of each base of the to-be-verified candidate multi-copy region is counted. For statistical results, see the number of multiple copies in the second row in the figure. By combining the values of the copy numbers of each position, the median value of the copy numbers of the candidate multi-copy regions can be obtained. The median value refers to the variable value positioned in the middle of a variable series that is formed by arranging the variable values in the statistical population in order of value size.

Further, in operation S100, the microorganism target fragment may be a chain or multiple incomplete motifs.

When the microorganism target fragment is multiple incomplete motifs, the motifs are connected together before searching for candidate multi-copy regions. There is no specific restriction on the order in which the motifs are connected together. The motifs may be connected in any order. For example, the motifs may be connected into a chain in random order. If a region where the similarity meets the preset value contains different motifs, the region is cut based on the original motif connection point and divided into two regions, to determine whether the two regions are candidate multi-copy regions, respectively.

The microorganism target fragment being multiple incomplete motifs means that part of the sequence of the microorganism target fragment is not a continuous single sequence, but is composed of multiple motifs of different sizes. The motif is caused by incomplete splicing of short read lengths under the existing second-generation sequencing conditions. This method is also suitable for whole genome sequence data generated by new technologies such as third-generation sequencing.

The microorganism target fragments in operation S1 are all derived from public databases, which are mainly selected from NCBI (https://www.ncbi.nlm.nih.gov).

Further, the method for identifying multi-copy regions in microorganism target fragments includes the following operations: S101, aligning the selected adjacent microorganism target fragments in pairs; if the similarity after alignment is lower than the preset value, issuing an alarm and displaying the screening conditions corresponding to the target strain.

Abnormal data caused by human errors or other reasons can be filtered.

The method of the present disclosure is not limited to whether there is a whole genome sequence. Operational tasks can be submitted by providing the names of the target strain and comparison strain or by uploading sequence files locally. In terms of detection scope, the method for identifying multi-copy regions in microorganism target fragments may cover all pathogenic microorganisms, including but not limited to bacteria, virus, fungi, amoebas, cryptosporidia, flagellates, microsporidia, piroplasma, plasmodia, toxoplasmas, trichomonas and kinetoplastids.

In a preferred embodiment, in operation S200, a 95% confidence interval of the copy numbers of the candidate multi-copy region is calculated. The confidence interval refers to the estimated interval of the overall parameter constructed by the sample statistics, that is, the interval estimation of the overall copy numbers of the target region. The confidence interval reflects the degree to which the true value of the copy numbers of the target region has a certain probability to fall around the measurement result. The confidence interval gives the credibility of the measured value of the measured parameter.

When calculating the 95% confidence interval of the copy numbers of the candidate multi-copy region, the base number of the candidate multi-copy region serves as the sample number, and the copy numbers value corresponding to each base in the candidate multi-copy region serves as the sample value.

As shown in FIG. 1-2, in the multi-copy target region with a length of 500 bp, each base corresponds to one copy number value, then a set of 500 copy number values in total are located in the multi-copy target region.

In addition to the median value of the copy numbers mentioned above, the present disclosure uses the 95% confidence interval of these 500 copy number values to measure the interval estimation of the overall copy numbers of the multi-copy target region when the significance level is 0.05 and the confidence level is 95%. When the confidence level is the same, the more samples, the narrower the confidence interval, and the closer to the mean value.

The microorganism target fragment may be a whole genome of a microorganism or a gene fragment of a microorganism.

The mechanism of the present disclosure is that, under normal circumstances, the median value and 95% confidence interval representing these 500 copy number values can reflect the real condition of the candidate multi-copy region. In addition to further verifying the multiple copies, the design of the module can also exclude some special cases. For example, if only 5 bases in the 500-bp candidate multi-copy region have a copy number of 1000, and the remaining 495 bases have a copy number of 1, then in this case, the median value of the copy numbers is 1, but the mean value is 10.99, and the 95% confidence interval ranges from 2.25 to 19.73. Obviously, although the mean value indicates multiple copies, the median value is no longer within the 95% confidence interval. Therefore, the candidate multi-copy region cannot be judged as a multi-copy region.

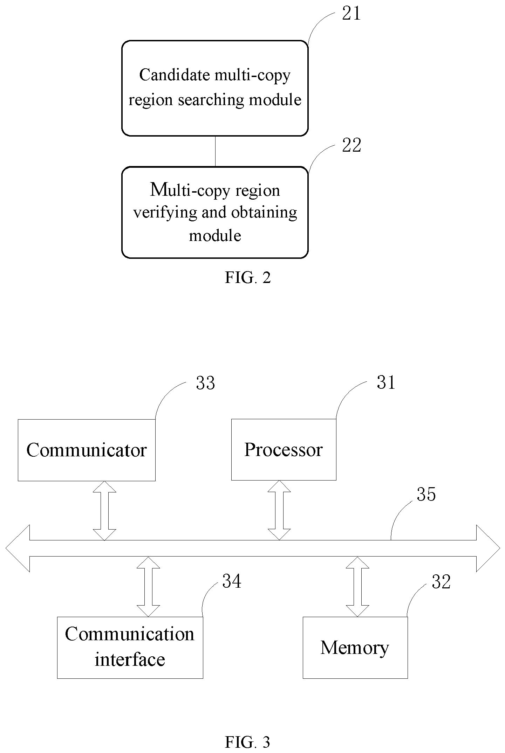

As shown in FIG. 2, the device for identifying multi-copy regions in microorganism target fragments according to the present disclosure includes at least a candidate multi-copy region searching module and a multi-copy region verifying and obtaining module.

The candidate multi-copy region searching module performs internal alignment on a microorganism target fragment, and searches for a region corresponding to a to-be-detected sequence of which a similarity meets a preset value as a candidate multi-copy region, the similarity is a product of a coverage rate and a matching rate of the to-be-detected sequence.

The multi-copy region verifying and obtaining module obtains a median value of copy numbers of the candidate multi-copy region. If the median value of the copy numbers of the candidate multi-copy region is greater than 1, the candidate multi-copy region is recorded as a multi-copy region.

The coverage rate = (length of similar sequence/(end value of the to-be-detected sequence - starting value of the to-be-detected sequence + 1))%.

The matching rate refers to the identity value when the to-be-detected sequence is aligned with another sequence. The identity value of the two aligned sequences may be obtained by software such as needle, water or blat.

The preset value of the similarity may be determined as needed. The recommended preset value of the similarity should exceed 80%, such as 85%, 90%, 95%, 96%, 97%, 98%, 99%, or 100%.

Further, the positions of the bases between the two to-be-aligned sequences do not cross.

Optionally, the candidate multi-copy region searching module further includes a raw data similarity comparison submodule, to align the selected adjacent microorganism target fragments in pairs; if the similarity after alignment is lower than the preset value, an alarm is issued and the screening conditions corresponding to the target strain are displayed. Users may re-select a target strain to enter the background calculation based on the feedback report.

In the candidate multi-copy region searching module, when the microorganism target fragment includes multiple incomplete motifs, the motifs are connected together before searching for candidate multi-copy regions.

If a region where the similarity meets the preset value contains different motifs, the region is cut based on the original motif connection point and divided into two regions, to determine whether the two regions are candidate multi-copy regions, respectively.

The motifs are connected in any order.

The multi-copy region verifying and obtaining module further includes a candidate multi-copy region copy number median value obtaining submodule, to determine the position of each candidate multi-copy region on the microorganism target fragments, obtain the number of other candidate multi-copy regions covering the position of each base of the to-be-verified candidate multi-copy region, and calculate the median value of the copy numbers of the to-be-verified candidate multi-copy region.

The multi-copy region verifying and obtaining module is further configured to calculate a 95% confidence interval of the copy numbers of the candidate multi-copy region.

When calculating the 95% confidence interval of the copy numbers of the candidate multi-copy region, the base number of the candidate multi-copy region serves as the sample number, and the copy number value corresponding to each base in the candidate multi-copy region serves as the sample value.

Since the principles of the device in the present embodiment is basically the same as that of the above-mentioned method embodiment, the definitions of the same features, the calculation methods, the enumeration of the embodiments, and the enumeration of the preferred embodiments may be used interchangeably, thus will not be described again.

It should be noted that the division of each module of the above apparatus is only a division of logical functions. In actual implementation, the modules may be integrated into one physical entity in whole or in part, or may be physically separated. These modules may all be implemented in the form of processing component calling by software. These modules may also be implemented entirely in hardware. It is also possible that some modules are implemented in the form of processing component calling by software, and some modules are implemented in the form of hardware. For example, the obtaining module may be a separate processing element, or may be integrated into a chip, or may be stored in a memory in the form of program code. The function of the above obtaining module is called and executed by one of the processing elements. The implementation of other modules is similar. In addition, all or part of these modules may be integrated or implemented independently. The processing elements described herein may be an integrated circuit with signal processing capabilities. In the implementation process, each operation of the above method or each of the above modules may be implemented by an integrated logic circuit of hardware in the processor element or instruction in a form of software.

For example, the above modules may be one or more integrated circuits configured to implement the above method, such as one or more application specific integrated circuits (ASIC), or one or more digital signal processors (DSP), or one or more field programmable gate arrays (FPGA) or graphics processing unit (GPU). As another example, when one of the above modules is implemented in the form of calling program codes of a processing element, the processing element may be a general processor, such as a central processing unit (CPU) or other processors that may call program codes. As another example, these modules may be integrated and implemented in the form of a system-on-a-chip (SOC).

Some embodiments of the present disclosure further provide a computer readable storage medium, which stores a computer program. When executed by a processor, the program implements the above-mentioned method for identifying multi-copy regions in microorganism target fragments.

Some embodiments of the present disclosure provide a computer processing device, including a processor and the above-mentioned computer readable storage medium. The processor executes the computer program on the computer readable storage medium to implement the operations of the above-mentioned method for identifying multi-copy regions in microorganism target fragments.

Some embodiments of the present disclosure provide an electronic terminal, including a processor, a memory and a communicator; the memory stores a computer program, the communicator communicates with an external device, and the processor executes the computer program stored in the memory, so that the electronic terminal executes and implements the above-mentioned method for identifying multi-copy regions in microorganism target fragments.

FIG. 3 is a schematic diagram showing the electronic terminal provided by the present disclosure. The electronic terminal includes a processor 31, a memory 32, a communicator 33, a communication interface 34 and a system bus 35. The memory 32 and the communication interface 34 are connected and communicated with the processor 31 and the communicator 33 through the system bus 35. The memory 32 is used to store computer programs. The communicator 33 and the communication interface 34 are used to communicate with other devices. The processor 31 and the communicator 33 are used to execute the computer programs, so that the electronic terminal performs the operations of the above method for identifying multi-copy regions in microorganism target fragments.

The system bus mentioned above may be a peripheral component interconnect (PCI) bus or an extended industry standard architecture (EISA) bus, etc. The system bus may include address bus, data bus, control bus and so on. For convenience of representation, only a thick line is used in the figure, but it does not mean that there is only one bus or one type of bus. The communication interface is used to implement communication between the database access device and other devices (such as a client, a read-write library, and a read-only library). The memory 301 may include a random access memory (RAM), or may also include a non-volatile memory, such as at least one disk memory.

The above-mentioned processor may be a general processor, including a central processing unit (CPU), a network processor (NP), and the like. The above-mentioned processor may also be a digital signal processor (DSP), an application specific integrated circuit (ASIC), a field-programmable gate array (FPGA), a graphics processing unit (GPU) or other programmable logic devices, discrete gate or transistor logic devices, or discrete hardware components.

Those of ordinary skill will understand that all or part of the operations to implement the various method embodiments described above may be accomplished by hardware associated with a computer program. The above-mentioned computer programs may be stored in a computer readable storage medium. The program, when executed, performs the operations including the above method embodiments. The computer readable storage mediums may include, but are not limited to, floppy disks, optical disks, compact disc read-only memories (CD-ROM), magneto-optical disks, read only memories (ROM), random access memories (RAM), erasable programmable read-only memory (EPROM), electrically erasable programmable read-only memory (EEPROM), magnetic cards or optical cards, flash memories, or other types of medium or machine-readable media suitable for storing machine-executable instructions. The computer readable storage medium may be a product that is not accessed to a computer device, or a component that has been accessed to a computer device for use.

In terms of specific implementation, the computer programs may be routines, programs, objects, components, data structures or the like that perform specific tasks or implement specific abstract data.

The above-mentioned method for identifying multi-copy regions in microorganism target fragments, the device for identifying multi-copy regions in microorganism target fragments, the computer readable storage medium, the computer processing device or the electronic terminal may be used in PCR detection of microorganisms, and specifically, in screening of template sequences.

The above-mentioned device for identifying multi-copy regions in microorganism target fragments, the above-mentioned computer readable storage medium, the above-mentioned computer processing device or the above-mentioned electronic terminal may be used for detecting multi-copy regions in microorganism target fragments.

The microorganism may be selected from one or more of bacterium, virus, fungus, amoeba, cryptosporidium, flagellate, microsporidium, piroplasma, plasmodium, toxoplasma, trichomonas and kinetoplastid.

The above-mentioned embodiments are merely illustrative of the principle and effects of the present disclosure instead of limiting the present disclosure. Modifications or variations of the above-described embodiments may be made by those skilled in the art without departing from the spirit and scope of the disclosure. Therefore, all equivalent modifications or changes made by those who have common knowledge in the art without departing from the spirit and technical concept disclosed by the present disclosure shall be still covered by the claims of the present disclosure.

**Claims**

1. A method for identifying a multi-copy region in a microorganism target fragment, comprising at least the following operations:

   S100, searching for a candidate multi-copy region: performing an internal alignment on a microorganism target fragment, and searching for a region corresponding to a to-be-detected sequence of which a similarity meets a preset value as the candidate multi-copy region, the similarity being a product of a coverage rate and a matching rate of the to-be-detected sequence;
   S200, verifying and obtaining the multi-copy region: obtaining a median value of copy numbers of the candidate multi-copy region; if the median value of the copy numbers of the candidate multi-copy region is greater than 1, the candidate multi-copy region is recorded as a multi-copy region.

2. The method for identifying a multi-copy region in a microorganism target fragment according to claim 1, further comprising one or more of the followings:

a.

$$\text{the coverage rate} = (\text{length of similar sequence} / (\text{end value of the to-be-detected sequence} - \text{starting value of the to-be-detected sequence} + 1))\%;$$

b. the preset value of similarity exceeds 80%;

c. positions of bases between two to-be-aligned sequences do not cross;

d. the method further comprises the following operations: S101, aligning selected adjacent microorganism target fragments in pairs; if the similarity after alignment is lower than the preset value, issuing an alarm and displaying screening conditions corresponding to a target strain;

e. in operation S200, a 95% confidence interval of the copy numbers of the candidate multi-copy region is calculated.

3. The method for identifying a multi-copy region in a microorganism target fragment according to claim 2, wherein when calculating the 95% confidence interval of the copy numbers of the candidate multi-copy region, a base number of the candidate multi-copy region serves as a sample number, and a copy number value corresponding to each base in the candidate multi-copy region serves as a sample value for calculation.

4. The method for identifying a multi-copy region in a microorganism target fragment according to claim 1, wherein when the microorganism target fragment includes multiple incomplete motifs, the motifs are connected together before searching for the candidate multi-copy region.

5. The method for identifying a multi-copy region in a microorganism target fragment according to claim 3, further comprising one or more of the followings:

a. if a region where the similarity meets the preset value contains different motifs, the region is cut based on an original motif connection point and divided into two regions, to determine whether the two regions are candidate multi-copy regions, respectively;

b. the motifs are connected in any order.

6. The method for identifying a multi-copy region in a microorganism target fragment according to claim 1, wherein the obtaining of the median value of the copy numbers of the candidate multi-copy region includes: determining a position of each candidate multi-copy region on the microorganism target fragment, obtaining a number of other candidate multi-copy regions covering a position of each base of the to-be-verified candidate multi-copy region, and calculating the median value of the copy numbers of the to-be-verified candidate multi-copy region.

7. A device for identifying a multi-copy region in a microorganism target fragment, comprising at least the followings:

a candidate multi-copy region searching module, configured to perform internal alignment on a microorganism target fragment, and search for a region corresponding to a to-be-detected sequence of which a similarity meets a preset value as a candidate multi-copy region, the similarity being a product of a coverage rate and a matching rate of the to-be-detected sequence;

a multi-copy region verifying and obtaining module, configured to obtain a median value of copy numbers of the candidate multi-copy region; if the median value of the copy numbers of the candidate multi-copy region is greater than 1, the candidate multi-copy region is recorded as a multi-copy region.

8. The device for identifying a multi-copy region in a microorganism target fragment according to claim 7, further comprising one or more of the followings:

a.

$$\text{the coverage rate} = (\text{length of similar sequence}/ (\text{end value of the}$$

$$\text{to-be-detected sequence} - \text{starting value of the to-be-detected sequence} +$$

$$1))\%;$$

b. the preset value of similarity exceeds 80%;

c. positions of bases between two to-be-aligned sequences do not cross;

d. the candidate multi-copy region searching module further includes the following submodules: a raw data similarity comparison module, configured to align selected adjacent microorganism target fragments in pairs; if the similarity after alignment is lower than the preset value, an alarm is issued and screening conditions corresponding to a target strain are displayed;

e. the multi-copy region verifying and obtaining module is further configured to calculate a 95% confidence interval of the copy numbers of the candidate multi-copy region.

9. The device for identifying a multi-copy region in a microorganism target fragment according to claim 8, wherein when calculating the 95% confidence interval of the copy numbers of the candidate multi-copy region, a base number of the candidate multi-copy region serves as a sample number, and a copy number value corresponding to each base in the candidate multi-copy region serves as a sample value for calculation.

10. The device for identifying a multi-copy region in a microorganism target fragment according to claim 7, wherein in the candidate multi-copy region searching module, when the microorganism target fragment includes multiple incomplete motifs, the motifs are connected together before searching for the candidate multi-copy region.

11. The device for identifying a multi-copy region in a microorganism target fragment according to claim 10, further comprising one or more of the followings:

a. if a region where the similarity meets the preset value contains different motifs, the region is cut based on an original motif connection point and divided into two regions, to determine whether the two regions are candidate multi-copy regions, respectively;

b. the motifs are connected in any order.

12. The device for identifying a multi-copy region in a microorganism target fragment according to claim 7, wherein the multi-copy region verifying and obtaining module further includes a candidate multi-copy region copy number median value obtaining submodule, to determine a position of each candidate multi-copy region on the microorganism target fragment, obtain a number of other candidate multi-copy regions covering a position of each base of the to-be-verified candidate multi-copy region, and calculate the median value of the copy numbers of the to-be-verified candidate multi-copy region.

13. A computer readable storage medium, which stores a computer program, wherein when executed by a processor, the program implements the method for identifying a multi-copy region in a microorganism target fragment according to any one of claims 1-6.

14. A computer processing device, comprising a processor and the computer readable storage medium according to claim 13, wherein the processor executes the computer program on the computer readable storage medium to implement the operations of the method for identifying a multi-copy region in a microorganism target fragment according to any one of claims 1-6.

15. An electronic terminal, comprising a processor, a memory and a communicator; wherein the memory stores a computer program, the communicator communicates with an external device, and the processor executes the computer program stored in the memory, so that the electronic terminal executes the method for identifying a multi-copy region in a microorganism target fragment according to any one of claims 1-6.

16. A use of the method for identifying a multi-copy region in a microorganism target fragment according to any one of claims 1-6, the device for identifying a multi-copy region in a microorganism target fragment according to any one of claims 7-12, the computer readable storage medium according to claim 13, the computer processing device according to claim 14, or the electronic terminal according to claim 15 in the detection of a multi-copy region in a microorganism target fragment.

17. The use according to claim 16, wherein the microorganism includes one or more of bacterium, virus, fungus, amoeba, cryptosporidium, flagellate, microsporidium, piroplasma, plasmodium, toxoplasma, trichomonas, and kinetoplastid.

S1

Searching for a candidate multi-copy region: performing an internal alignment on a microorganism target fragment, and searching for a region corresponding to a to-be-detected sequence of which a similarity meets a preset value as the candidate multi-copy region, the similarity being a product of a coverage rate and a matching rate of the to-be-detected sequence

S2

Verifying and obtaining a multi-copy region: obtaining a median value of the copy number of the candidate multi-copy region; if the median value of the copy number of the candidate multi-copy region is greater than 1, the candidate multi-copy region is recorded as a multi-copy region

Start

End

FIG. 1

| Sequence 1 | Sequence 2 | Sequence matching rate | Alignment length | Mismatch length | Length of gap openings | Starting value of sequence 1 | End value of sequence 1 | Starting value of sequence 2 | End value of sequence 2 | e value | Score |
|---|---|---|---|---|---|---|---|---|---|---|---|
| Query id | Subject id | % identity | alignment length | mismatches | gap openings | q. start | q. end | s. start | s. end | e-value | bit score |
| A | B | 98.4 | 187 | 3 | 0 | 1 | 187 | 1170 | 1356 | 1.90E-98 | 356 |
| Sequence coverage rate = (alignment length /(end value of sequence 1- starting value of sequence 1 + 1)) | | | | | | | | | | | |
| Similarity = sequence coverage rate * sequence matching rate | | | | | | | | | | | |

FIG. 1-1

Whole genome sequence:

-----CGCATTTCGATTTCAGCAGGAATACCTTCTACTAAATCATTGCAAAGTTTTTCAAAGGCAAAGAGA-------CAATCTTTTCTTGAATTTCTAGAGGTGGAAAAGAGAGTTCAATTTCAGCCATTACTTTTTGGCTTAGTTTTGGTCTTG------

Repeat region

Number of multiple copies:

5566666666655667777777777677------6665555566666655555444444444

Repetitive fragment

FIG. 1-2

21

Candidate multi-copy
region searching module

22

Multi-copy region
verifying and obtaining
module

FIG. 2

33

Communicator

31

Processor

35

34

Communication
interface

32

Memory

FIG. 3

<div align="center">

## INTERNATIONAL SEARCH REPORT

</div>

| | International application No. |
|---|---|
| | **PCT/CN2020/090175** |

**A. CLASSIFICATION OF SUBJECT MATTER**

G16B 30/10(2019.01)i

According to International Patent Classification (IPC) or to both national classification and IPC

**B. FIELDS SEARCHED**

Minimum documentation searched (classification system followed by classification symbols)

G16B; G06F

Documentation searched other than minimum documentation to the extent that such documents are included in the fields searched

Electronic data base consulted during the international search (name of data base and, where practicable, search terms used)

WPI, EPODOC, CNPAT, CNKI: 基因, 序列, 拷贝, 测序, 覆盖率, 识别, 检测, 匹配, 筛选, 片段, copies, similar+, compar+, match+, genic

**C. DOCUMENTS CONSIDERED TO BE RELEVANT**

| Category* | Citation of document, with indication, where appropriate, of the relevant passages | Relevant to claim No. |
|---|---|---|
| A | CN 101930502 A (BGI SHENZHEN CO., LIMITED) 29 December 2010 (2010-12-29) description, paragraphs [0026]-[0091], and figures 1-8 | 1-17 |
| A | CN 104870056 A (GENENTECH INC.) 26 August 2015 (2015-08-26) entire document | 1-17 |
| A | CN 103810402 A (BEIJING NOVOGENE BIOINFORMATICS TECHNOLOGY CO., LTD.) 21 May 2014 (2014-05-21) entire document | 1-17 |
| A | US 6694335 B1 (MICROSOFT CORPORATION) 17 February 2004 (2004-02-17) entire document | 1-17 |

☐ Further documents are listed in the continuation of Box C.   ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **23 December 2020** | **31 December 2020** |

| Name and mailing address of the ISA/CN | Authorized officer |
|---|---|
| **China National Intellectual Property Administration (ISA/ CN)** **No. 6, Xitucheng Road, Jimenqiao, Haidian District, Beijing 100088** **China** | |
| Facsimile No. **(86-10)62019451** | Telephone No. |

Form PCT/ISA/210 (second sheet) (January 2015)

**INTERNATIONAL SEARCH REPORT**
**Information on patent family members**

International application No.

**PCT/CN2020/090175**

| Patent document cited in search report | | | Publication date (day/month/year) | Patent family member(s) | | | Publication date (day/month/year) |
|---|---|---|---|---|---|---|---|
| CN | 101930502 | A | 29 December 2010 | HK | 1148372 | A1 | 09 November 2012 |
| CN | 104870056 | A | 26 August 2015 | CA | 2884368 | A1 | 10 April 2014 |
| | | | | MX | 2015003932 | A | 23 July 2015 |
| | | | | JP | 2020120656 | A | 13 August 2020 |
| | | | | US | 2015307611 | A1 | 29 October 2015 |
| | | | | IL | 269529 | D0 | 28 November 2019 |
| | | | | WO | 2014055824 | A1 | 10 April 2014 |
| | | | | PL | 2903691 | T3 | 31 October 2019 |
| | | | | EP | 2903691 | A1 | 12 August 2015 |
| | | | | RU | 2015112024 | A | 27 November 2016 |
| | | | | AR | 092908 | A1 | 06 May 2015 |
| | | | | ES | 2738305 | T3 | 21 January 2020 |
| | | | | JP | 2016501510 | A | 21 January 2016 |
| | | | | US | 2018346576 | A1 | 06 December 2018 |
| | | | | KR | 20150058270 | A | 28 May 2015 |
| | | | | JP | 2018085989 | A | 07 June 2018 |
| | | | | AU | 2013326921 | A1 | 02 April 2015 |
| | | | | HK | 1213522 | A0 | 08 July 2016 |
| | | | | SG | 11201502548 | A1 | 29 April 2015 |
| | | | | BR | 112015007528 | A2 | 04 July 2017 |
| | | | | IL | 237650 | A | 31 March 2020 |
| CN | 103810402 | A | 21 May 2014 | None | | | |
| US | 6694335 | B1 | 17 February 2004 | None | | | |

Form PCT/ISA/210 (patent family annex) (January 2015)